# EUROPÄISCHE PATENTANMELDUNG

(11) **EP 0 525 480 A1**
(43) Veröffentlichungstag der Anmeldung: **03.02.1993**
(21) Anmeldenummer: 92111799.0
(22) Anmeldetag: 10.07.1992
(51) Int. Cl.: A61F 2/46

(54) **Steril-Hülse**

(30) Priorität: 25.07.1991 DE 9109207 U
(71) Anmelder: THERA Patent GmbH & Co. KG Gesellschaft für industrielle Schutzrechte, D-82229 Seefeld (DE)
(72) Erfinder: Brandhorst, Gerd, Dipl.-Ing., W-8000 München 71 (DE)
(74) Vertreter: Strehl Schübel-Hopf Groening & Partner

(57) **Zusammenfassung**

Der beschriebene Behälter schützt eine sterile Kapsel mit vordosiertem, mehrkomponentigen härtbaren Material, beispielsweise Knochenzement, vor Keimen und Verunreinigungen. Er ist in der Lage, einen von außen angewendeten Druck auf die Kapsel zu übertragen. Dabei kommen die in der Kapsel befindlichen Materialkomponenten miteinander in Kontakt und können durch Schütteln in einem oszillierenden Mischer miteinander vermischt werden. Auch wenn der Mischer nicht in einem sterilen Raum betrieben wird, bleibt die Kapsel dennoch keimfrei, was bei der Anwendung beispielsweise im Operationssaal wichtig ist.

Zur Übertragung des zur Aktivierung der Kapsel notwendigen Drucks von außen, besteht der Behälter aus zwei Schalenhälften 1 und 2, die bei Anwendung äußerer Druckkräfte zusammenschiebbar sind. Vorteilhafterweise ist die Kapsel beim Öffnen entweder durch einen Ring 3 oder oder durch einen Schirm vor Kontakt mit nicht-sterilen Flächen des Behälters geschützt.

## Beschreibung

Die Erfindung betrifft eine Hülse bzw. einen Behälter zur Aufnahme einer durch Druck aktivierbaren sterilen Kapsel für härtbares Material.

Derartige Kapseln werden beispielsweise im Dentalbereich verwendet. Sie enthalten vordosierte Materialkomponenten zur Herstellung eines gebrauchsfertigen Zements oder Füllmaterials, etwa eines Kunststoffs oder eines Amalgams.

Eine typische solche Kapsel ist in EP-A-0157121 beschrieben. Sie enthält eine pulverförmige Substanz sowie in einem Folienkissen eine flüssige Komponente. Bei der Anwendung wird mittels einer Zange Druck auf die Kapsel ausgeübt, so daß das Folienkissen zerreißt und die flüssige Komponente in Kontakt mit der pulverförmigen Substanz kommt. Beide Komponenten werden dann in einem Schüttler vermischt und dadurch zur Reaktion gebracht. Das fertig gemischte und damit gebrauchsfähige Präparat kann dann direkt an die zu behandelnde Stelle, beispielsweise in eine Zahnkavität gebracht werden, wo es aushärtet.

Die Anwendung der beschriebenen Kapsel hat den Vorteil, daß das vorgeschriebene Mischungsverhältnis der Komponenten stets exakt eingehalten wird. Dadurch sind die Aushärtezeit und die Eigenschaften des gehärteten Materials stets konstant und genau bekannt.

Das Anwendungsgebiet solcher Kapseln beschränkt sich bisher im wesentlichen auf die Dentalmedizin, wo keine besonderen Anforderungen an die Keimfreiheit der äußeren Kapseloberfläche gestellt werden.

Auf anderen medizinischen Gebieten besteht jedoch ebenfalls ein großer Bedarf an härtbaren Materialien, deren Komponenten exakt dosiert werden müssen. Dies gilt insbesondere für die Chirurgie, in der beim Implantieren von Prothesen usw. häufig Knochenzement benötigt wird.

Elektrische Mischer sind jedoch in der Regel zum Einsatz im Operationssaal nicht geeignet, da sie sich nicht beliebig oft sterilisieren lassen. Sowohl eine Wärmebehandlung als auch eine chemische Sterilisation würden dem Motor oder der Ansteuerelektronik Schaden zufügen. Auch die Möglichkeit, den Mischer außerhalb des Operationssaals aufzustellen und die Kapsel nach dem Mischvorgang, bevor sie in den Operationssaal gegeben wird, zu sterilisieren scheidet wegen der bis zum Aushärten des Zements zur Verfügung stehenden kurzen Zeitspanne aus.

Aus EP-A-0040959 ist ein Behälter mit einer darin angeordneten Kapsel bekannt, die eine Flüssigkeit zum Mischen mit einem in einer anderen Kammer des Behälters vorhandenen Pulver enthält. Der Behälter umfaßt ein zylindrisches Gefäß und eine Kappe mit angeformten Fingern und ist längs der Behälterachse derart verschiebbar, daß die Finger zwischen die Kapsel und die Behälter-Innenwand gezwängt werden, um die Kapsel zu zerbrechen und die Flüssigkeit in Kontakt mit dem Pulver zu bringen. Sodann werden die beiden Komponenten durch Schütteln vermischt und die Mischung ausgebracht. Dieser Behälter ist zum Einsatz in herkömmliche Mischgeräte weder bestimmt noch geeignet. Ferner ist eine Entnahme der Kapsel aus dem Behälter nicht vorgesehen; vielmehr wird die zerbrochene Kapsel mit dem Behälter nach Entleerung weggeworfen. Maßnahmen, die die Sterilität des Behälterinhalts nach dessen Öffnen gewährleisten, sind nicht vorhanden.

Aufgabe der Erfindung ist es daher, eine Vorrichtung zu schaffen, die den Einsatz der eingangs beschriebenen Kapsel in einer Umgebung mit hohen Anforderungen an die Keimfreiheit ermöglicht. Dabei sollen die konventionellen Druckzangen und Mischer verwendet werden können.

Diese Aufgabe wird mit einem Behälter nach Anspruch 1 gelöst. Dieser Behälter nimmt die vollständig, das heißt auch außen sterilisierte Kapsel auf und schützt sie vor Keimen und Verunreinigungen. Dadurch kann die Kapsel in dem Behälter auch in einer nicht sterilen Umgebung bequem gehandhabt werden. Der Behälter läßt sich so dünnwandig ausführen, daß er in den sonst für die Kapsel ohne Hülse bemessenen Halterungen der konventionellen oszillierenden Mischer Platz findet. Außerdem überträgt er dabei die Druckbewegung der Zange oder des Aktivators auf die Kapsel.

Druckzange und Mischer können außerhalb des Operationssaals betrieben werden. Nach erfolgter Mischung wird die sterile Kapsel in einer Schleuse dem Behälter entnommen und steril in den Operationssaal gebracht.

Die Ausgestaltungen nach den Ansprüchen 2 und 3 haben den Vorteil, daß die Kapsel beim Öffnen des Behälters besonders gut vor Kontakt mit nicht sterilen Flächen des Behälters geschützt ist. Die Ausgestaltung nach Anspruch 2 ist besonders einfach, da sie mit zwei Schalenteilen auskommt. Der an einem der Schalenteile angeformte Schirm verringert jedoch den für die Kapsel zur Verfügung stehenden Raum.

Diesen Nachteil vermeidet die Ausgestaltung nach Anspruch 3 dadurch, daß zwischen den Schalenteilen ein Ring vorgesehen ist, der bewirkt, daß diejenige Stelle, an der sich die beiden Behälterhälften unter Druckanwendung gegeneinander verschieben, um den Druck auf die innenliegende Kapsel zu übertragen, nicht identisch mit derjenigen Stelle ist, an der der Behälter geöffnet wird. So wird verhindert, daS die Kapsel beim Öffnen des Behälters in Kontakt mit einer Fläche eines Schalenteils gelangen kann, die beim Zusammendrücken des Behälters auf der Außenfläche des anderen Schalenteils gleitet und dabei Keime aufnimmt.

Bei dem Behälter nach Anspruch 4 ist auf besonders zweckmäßige Weise dafür gesorgt, daß der Ring beim Zusammenschieben des Behälters mit dem einen und beim Auseinanderziehen mit dem anderen Schalenteil verbunden bleibt.

Die Ausgestaltung nach Anspruch 5 ermöglicht eine bequeme Handhabung des Behälters beim Entnehmen der Kapsel.

Schnittbilder bevorzugter Ausführungsbeispiele der Erfindung sind in den Zeichnungen dargestellt. Darin zeigt
Figur 1 einen Behälter vor dem Einsetzen der Kapsel,
Figur 2 denselben Behälter in geschlossenem Zustand, wobei die innenliegende Kapsel aus Gründen der Übersichtlichkeit nicht dargestellt ist,
Figur 3 den wieder geöffneten Behälter, aus dem die Kapsel entnommen ist,
Figur 4 schematisch die Entnahme einer Kapsel aus einem Behälter mit Klemmflächen, und
Figur 5 ein mit einem Schirm versehenen Behälter gemäß einer anderen Ausführungsform.

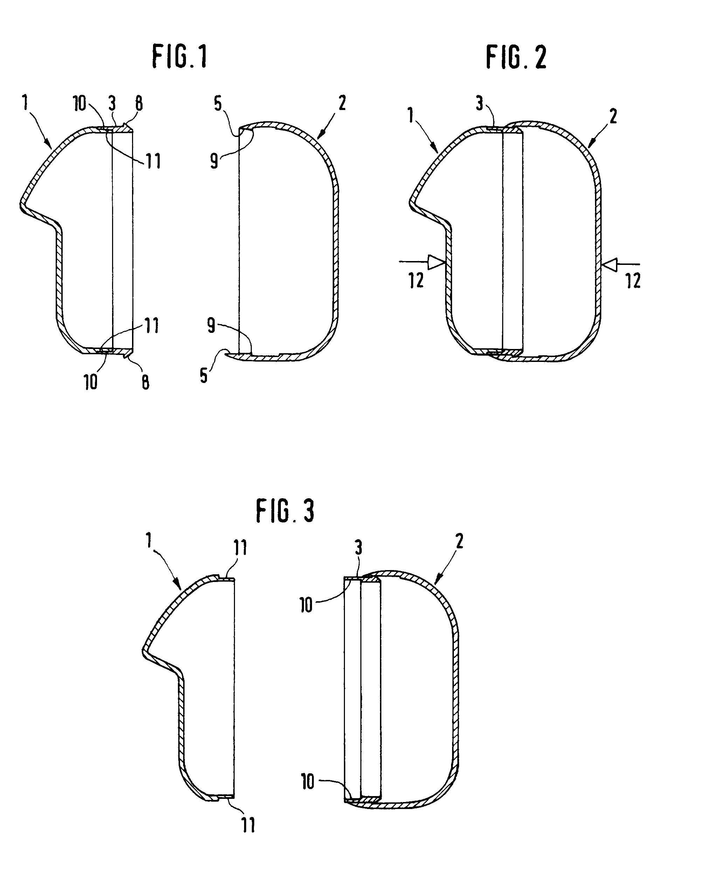

Der in Figur 1 im Schnitt abgebildete Behälter befindet sich im geöffneten Zustand vor dem Einsetzen der Kapsel. Er besteht aus zwei Schalenteilen 1 und 2 sowie aus einem dazwischen liegenden Ring 3. Größe und Form des Innenraums entsprechen genau der (aus Gründen der Übersichtlichkeit nicht dargestellten) Kapsel, so daß diese von dem Behälter dicht umschlossen wird. Die Teilungsebene der Schalenteile 1, 2 liegt im Bereich der Kapsel, so daß diese beim Öffnen des Behälters freiliegt.

Der Ring 3 ist auf das linke Schalenteil 1 aufgesteckt. Der Ring 3 und das linke Schalenteil 1 sind jeweils mit umlaufenden stufenförmigen Abschnitten 10 und 11 versehen, wobei der stufenförmige Abschnitt 10 des Rings 3 außen auf dem entsprechenden Abschnitt 11 des linken Schalenteils 1 sitzt.

An seiner rechten Außenkante ist an dem Ring 3 eine umlaufende Nase 8 angeformt, die hinter eine entsprechende Nase 9 des rechten Schalenteils 2 greift, wenn der Behälter wie in Figur 2 gezeigt geschlossen wird. Statt umlaufender Nasen 8 und 9 können auch einzelne Nasen vorgesehen sein.

In geschlossenem Zustand überlappt das rechte Schalenteil 2 außen den Ring 3, der wiederum im Bereich der stufenförmigen Abschnitte 10 und 11 das linke Schalenteil 1 außen überlappt. Dadurch läßt sich auf beiden Seiten des Rings 3 eine gleichmäßig wirkende Dichtkraft erzielen. Der Ring 3 kann aus dem Material eines der beiden Schalenteile 1 oder 2 bestehen, soll jedoch vorteilhafterweise elastischer als die beiden Schalenteile 1 und 2 sein. Außerdem sind die Durchmesser der Teile so gewählt, daß ein leichter Druck auf die dichtenden Kontaktflächen entsteht.

Am zweckmäßigsten wird der verschlossene Behälter zusammen mit der innenliegenden Kapsel durch radioaktive Bestrahlung sterilisiert und dann in einem keimdichten Steril-Beutel gelagert, aus dem er erst kurz vor der Verwendung entnommen wird. Der Behälter hält für eine begrenzte aber ausreichende Zeit steril dicht.

Zum Aktivieren einer im Behälter befindlichen (nicht gezeigten) Kapsel, werden die beiden Schalenteile 1 und 2 in Richtung der Pfeile 12 in Figur 2 zusammengedrückt. Dabei schiebt sich das rechte Schalenteil 2 etwas über den Ring 3 und über das linke Schalenteil 1. Dadurch wird die Druckkraft auf die innenliegende Kapsel übertragen. Aufgrund der stufenförmigen Abschnitte 10 und 11 des Rings 3 und das linken Schalenteils 1 verschieben sich diese beiden Teile beim Aktivieren nicht gegeneinander.

Das Zusammenschieben der Behälterhälften hat zur Folge, daß die in Figur 1 mit 5 bezeichnete Dichtfläche des rechten Schalenteils 2 auf der Außenseite des Rings 3 bzw. des linken Schalenteils 1 gleitet. Dabei kommt die Fläche 5 in Kontakt mit auf der Außenseite des Behälters befindlichen Keimen und Verunreinigungen.

Zum Öffnen des Behältern werden die beiden Schalenteile auseinander gezogen und vollständig getrennt. Um die sterile Kapsel dabei vor Kontakt mit der nicht mehr sterilen Fläche 5 des rechten Schalenteils 2 zu schützen, läßt sich der Behälter nicht an der Verbindungsstelle zwischen Ring 3 und rechtem Schalenteil 2 öffnen, an der die gleitende, zusammenschiebende Bewegung stattgefunden hat. Vielmehr bewirken die Nasen 8 und 9 des Rings 3 und des rechten Schalenteils 2, daß diese beiden Teile miteinander verhakt bleiben. Beim Auseinanderziehen der Schalenteile 1, 2 öffnet sich der Behälter daher wie in Figur 3 gezeigt zwischen dem linken Schalenteil 1 und dem Ring 3. Der Ring 3 bleibt mit dem rechten Schalenteil 2 verbunden und deckt die nicht mehr sterile innere Dichtfläche 5 des rechten Schalenteils 2 ab.

Wie beschrieben ist der Ring 3 auf das linke Schalenteil 1 aufgesteckt und wird zum Öffnen des Behälters von diesem abgezogen. Beide Teile können jedoch auch lediglich aus einem einzigen Stück gefertigt sein und werden dann an einer aufzureißenden Stelle voneinander getrennt.

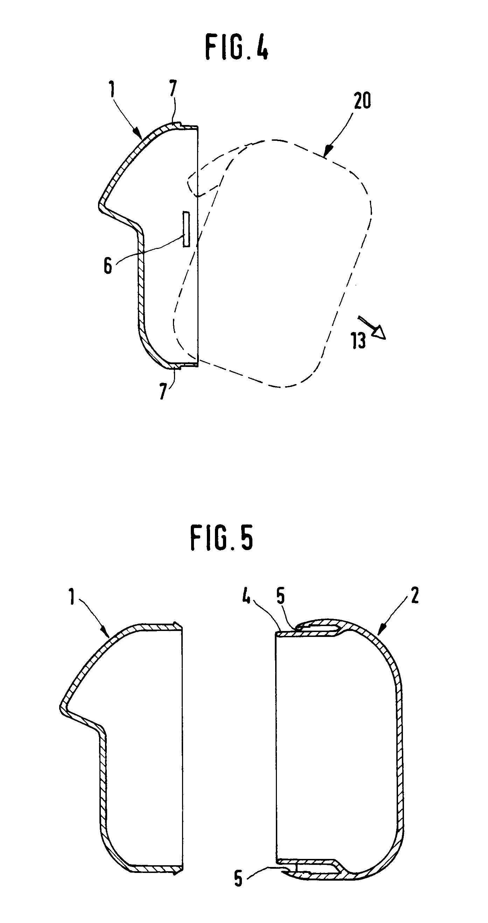

Das in Figur 4 dargestellte linke Schalenteil 1 ist an zwei gegenüberliegenden Innenseiten mit Klemmflächen 6 versehen, von denen im Schnittbild nur eine sichtbar ist. Zwischen den Klemmflächen 6 wird die Kapsel 20 ähnlich wie von einer Klammer gehalten, wenn der Behälter geöffnet wird. Durch Druck auf zwei andere, quer zu den Klemmflächen 6 liegende Flächen 7 des Schalenteils 1 deformiert sich dieses und die Wände, an deren Innenseite sich die Klemmflächen 6 befinden, wölben sich nach außen. Dadurch wird die Kapsel freigegeben und kann in Richtung des Pfeils 13 beispielsweise auf ein steriles Tablett fallen, ohne daß sie berührt zu werden braucht. Dies kann beispielsweise in der Schleuse zu einem Operationssaal geschehen. Auf dem Tablett wird die Kapsel dann in den Operationssaal hineingereicht.

Bei dem in Figur 5 dargestellten Behälter übernimmt ein an dem rechten Schalenteil 2 angeformter Schirm 4 die Schutzfunktion des oben beschriebenen Rings 3. Im übrigen entspricht dieser Behälter in seiner Anwendung dem oben beschriebenen Ausführungsbeispiel. Auch hier schieben sich die beiden Schalenteile 1 und 2 bei Druckanwendung zusammen. Dabei gleitet die nach innen weisende Dichtfläche 5 des rechten Schalenteils 2, das das andere Schalenteil 1 in zusammengesetztem Zustand außen überlappt, auf der Außenfläche des Behälters und kommt dabei in Kontakt mit Keimen. Beim Öffnen des Behälters trennen sich die beiden Schalenteile. Dabei wird die innenliegende (nicht dargestellte) Kapsel durch den Schirm 4 vor Kontakt mit der nicht mehr sterilen Dichtfläche 5 geschützt.

Das Vorhandensein des Schirms 4 bedingt, daß der im Inneren des Behälters für die Kapsel zur Verfügung stehende Raum etwas kleiner ist als bei dem Ausführungsbeispiel der Figuren 1 bis 4. Andererseits hat der Behälter nach der Figur 5 den Vorteil, daß die Zahl der Dichtflächen geringer ist und, insbesondere wenn der Schirm wie dargestellt einstückig mit dem rechten Schalenteil 2 ausgebildet ist, die Zahl der mit der entsprechenden Präzision zu fertigenden Teile geringer ist.

Bei den beschriebenen Ausführungsbeispielen wird der Behälter durch Auseinanderziehen der beiden Schalenteile 1 und 2 geöffnet, wobei diese sich vollständig voneinander trennen. Es ist jedoch auch denkbar, daß sie durch ein Scharnier oder einen biegsamen Streifen verbunden bleiben.

Die Hülse besteht vorzugsweise aus durchsichtigem Material, da dadurch die Art der in ihrem Inneren befindlichen Kapsel von außen erkennbar ist. Die Kapsel ist dazu zweckmäßigerweise mit einer Farbcodierung versehen.

## Patentansprüche

1. Behälter zur Aufnahme einer durch Druck aktivierbaren sterilen Kapsel (20) für härtbares Material,
gekennzeichnet durch
zwei steril dichtende Schalenteile (1,2), die sich bei Druckanwendung zusammenschieben und dabei den Druck auf die innenliegende Kapsel (20) übertragen und die zur anschließenden Entnahme der Kapsel (20) zu öffnen sind, und
eine Einrichtung (3, 4), die die Kapsel (20) beim Öffnen der Schalenteile (1, 2) vor Kontakt mit derjenigen Fläche (5) eines Schalenteils (2) schützt, die beim Zusammenschieben auf der Außenfläche des anderen Schalenteils (1) gleitet.

2. Behälter nach Anspruch 1, dadurch gekennzeichnet, daß die Schutzeinrichtung ein an dem besagten einen Schalenteil (2) angebrachter Schirm (4) ist, der über den Rand dieses Schalenteils hinausragt und im zusammengeschobenen Zustand zwischen dem anderen Schalenteil (1) und der Kapsel zu liegen kommt.

3. Behälter nach Anspruch 1, dadurch gekennzeichnet, daß die Schutzeinrichtung ein zwischen den Schalenteilen (1, 2) vorgesehener ein Ring (3) ist, der beim Zusammenschieben der Schalenteile (1, 2) mit dem ersten Schalenteil (1) starr und mit dem zweiten verschiebbar verbunden ist, während er sich beim Öffnen der Schalenteile (1, 2) von dem ersten Schalenteil (1) löst und mit dem zweiten Schalenteil (2) verbunden bleibt.

4. Behälter nach Anspruch 3, dadurch gekennzeichnet, daß der Ring (3) und das zweite Schalenteil (2) jeweils mit zusammenwirkenden Nasen (8,9) versehen sind, die einerseits beim Zusammenschieben ein Gleiten dieser beiden Teile gegeneinander zulassen, andererseits beim Auseinanderziehen und Öffnen der beiden Schalenteile (1, 2) bewirken, daß der Ring (3) und das zweite Schalenteil (2) miteinander verhaken.

5. Behälter nach einem der Ansprüche 1 bis 4, dadurch gekennzeichnet, daß die Schalenteile (1, 2) beim Öffnen völlig voneinander trennbar sind, daß eines der Schalenteile (1) an seiner Innenseite mit zwei einander gegenüberliegenden Klemmflächen (6) versehen ist, zwischen denen die Kapsel (20) klemmt, und daß dieses Schalenteil nach dem Öffnen des Behälters durch Druck auf zwei Außenflächen (7) deformierbar ist, so daß die Spannung von den Klemmflächen (6) gelöst und die Kapsel (20) freigegeben wird.
